(19) Europäisches Patentamt
European Patent Office
Office européen des brevets

(11) Publication number: **0 055 630**
**B1**

(12) # EUROPEAN PATENT SPECIFICATION

(45) Date of publication of patent specification: **18.12.85**

(51) Int. Cl.[4]: **C 07 D 209/48,**
C 07 D 307/83, C 07 C 103/24

(21) Application number: **81306178.5**

(22) Date of filing: **30.12.81**

(54) **Method for the preparation of fluorophthalamic compounds.**

(30) Priority: **29.12.80 US 220672**
**29.12.80 US 220674**
**26.01.81 US 228656**
**29.12.80 US 220664**
**29.12.80 US 220673**

(43) Date of publication of application:
**07.07.82 Bulletin 82/27**

(45) Publication of the grant of the patent:
**18.12.85 Bulletin 85/51**

(84) Designated Contracting States:
**BE DE FR GB IT NL**

(56) References cited:
**EP-A-0 026 749**

**THE JOURNAL OF ORGANIC CHEMISTRY, vol. 42, no. 17, August 19, 1977 WASHINGTON (US) R.L. MARKEZICH et al.: "Reactions of 4-nitrophthalic anhydride with potassium fluoride and potassium nitrite" pages 3435-3436**

(73) Proprietor: **OCCIDENTAL CHEMICAL CORPORATION**
**P.O. Box 189**
**Niagara Falls New York 14302 (US)**

(72) Inventor: **Fifolt, Michael J.**
**2225 Staley Road**
**Grand Island New York 14072 (US)**
Inventor: **Foster, Arthur M.**
**32870 Robin Hood**
**Birmingham Michigan 48010 (US)**
Inventor: **Cotter, Byron R.**
**262 Laurie Lane**
**Grand Island New York 14072 (US)**

(74) Representative: **Myerscough, Philip Boyd et al**
**J.A.Kemp & Co. 14, South Square Gray's Inn**
**London, WC1R 5EU (GB)**

**EP 0 055 630 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new fluorophthalamic compounds and to a process for their preparation. Novel ammonium fluorophthalamates, fluorophthalamic acids, fluorophthalimides, fluorophthaloyl fluoride, and alkali metal salts of fluorophthalimides can be prepared through the process of the invention and are particularly useful as chemical intermediates for the preparation of a wide variety of end products including pharmaceuticals and agricultural chemicals such as pecticides, and, in particular, as reactants in novel and advantageous methods for the synthesis of fluoroanthranilic acids, fluoroanilines, fluorobenzoic acids and other fluorinated aromatic compounds.

In accordance with this invention there are provided novel fluorophthalamic compounds of the formula

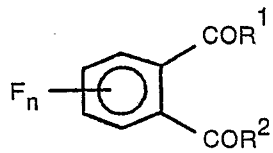

wherein n is 1 or 2, and $R^1$ is $-NH_2$ and $R^2$ is $-OH$ or $-ONH_4$ or $R^1$ and $R^2$ together are

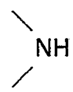 or 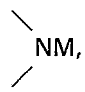,

wherein M is an alkali metal.

The invention also provides a process for preparing an ammonium salt of fluorophthalamic acid of the formula

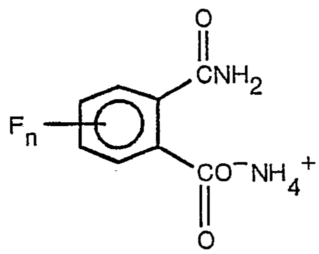

wherein n is 1 or 2, which process comprises

A) reacting a chlorophthalic anhydride of the formula

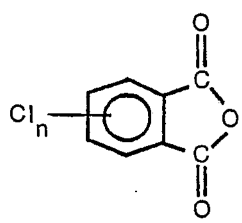

wherein n is 1 or 2, with potassium fluoride or cesium fluoride to form a fluorophthalic anhydride of the formula

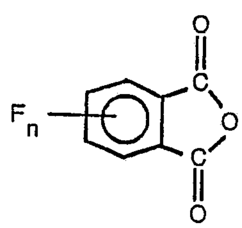

where n is 1 or 2; and

B) reacting the fluorophthalic anhydride with ammonia to form an ammonium salt of a fluorophthalamic acid.

A fluorophthalamic acid of formula

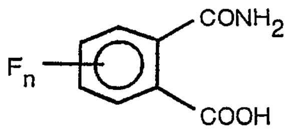

,

where n is 1 or 2, can then be prepared by (C) acidifying an ammonium fluorophthalamate resulting from process step (B).

A fluorophthalimide of formula

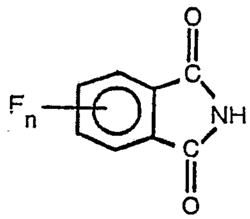

,

where n is 1 or 2, can then be prepared by (D) heating an ammonium fluorophthalamate resulting from process step (B) to 150 to 350°C, or by (D') dehydrating a fluorophthalamic acid resulting from process step (C).

A fluorophthalimide resulting from process step (D) or (D') can be reacted with an alkali metal base to form an alkali metal salt of a fluorophthalimide of the formula

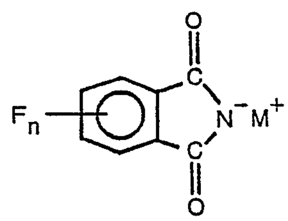

,

where n is 1 or 2 and M is an alkali metal.

(A) The fluorination step of the process, that is the preparation of fluorophthalic anhydride by reaction of chlorophthalic anhydride with potassium fluoride or cesium fluoride, may be carried out under a wide range of conditions. The temperature of the reaction may vary considerably for example, from 75° or lower to 300° Celsius or higher, depending, in part on the particular chlorophthalic anhydride reactant used and whether or not a catalyst is employed. The reaction is preferably carried out in the presence of a catalyst, at a temperature of 80° to 280° Celsius. When potassium fluoride is employed as the fluorinating agent, it is preferred to carry out the reaction at higher temperatures, such as 180° to 275° Celsius. When cesium fluoride is employed as the fluorinating agent, lower temperatures, such as 80° to 200° Celsius are preferred.

The preferred catalysts that may be employed in the fluorination step are polyether catalysts, such as crown ethers, polyethylene glycols, or alkoxy polyethylene glycols, such as polyethers typically having a molecular weight ranging from 200 to 25,000. Typically, the catalysts are employed in amounts of 0.1 to 20 percent, and preferably 5.0 to 15 percent by weight, based on the amount of chlorophthalic anydride.

The fluorination reaction is preferably and conveniently carried out under atmospheric pressure conditions. However, super-atmospheric and sub-atmospheric conditions may be employed, if desired.

It is preferred, but not essential, to employ a catalyst when potassium fluoride is used as the fluorinating agent. When cesium fluoride is used, a catalyst may be employed, but it is not preferred, due to the higher reactivity of that fluorinating agent.

Although it is preferred to run the fluorination reaction neat, an inert solvent, such as dipolar aprotic solvent may be employed if desired. Suitable solvents include, for example, dimethylsulfoxide; dimethylformamide; N-methyl-2-pyrrolidinone; sulfolane; and hexamethylphosphoramide.

The proportion of reactants for the fluorination step may vary widely. Generally it is preferred to employ 20 to 25 percent molar excess of potassium fluoride or cesium fluoride fluorinating agent.

If anhydrous potassium fluoride is utilized in the fluorination agent with 4,5-dichlorophthalic anhydride as a reactant and the molar ratio of potassium fluoride: dichlorophthalic anhydride maintained at from 4:1 to 20:1 the novel compound 4,5-difluorophthaloyl fluoride can be produced in economic quantities along with the fluorophthalic anhydride of the disclosed reaction sequence. 4,5-Difluorophthaloyl fluoride is useful as a chemical intermediate in the preparation of various chemical end products, such as 4,5-difluorophthalimide and resins such as polyamides. Polyamides having fluorine substituents on the polymer chain may be prepared by polycondensation of 4,5-difluorophthaloyl fluoride with a suitable diamine, such as piperazine, utilizing such known techniques as interfacial polymerization or solution polymerization.

(B) The ammonolysis of the fluorophthalic anhydride may be carried out over a wide range of temperatures. The preferred reaction temperature is −50° to 100°, and most preferably −10° to 40° Celsius. Lower temperatures may be employed but are generally unnecessary and uneconomical. At temperatures above 40° Celsius, depending on the particular fluorophthalamic acid being prepared, the concurrent formation of the fluorophthalimide may occur.

To achieve high yields of the ammonium fluorophthalamate it is preferred to employ the ammonia reactant in excess of about two molar equivalents of $NH_3$ per mile of fluorophthalic anhydride. The preferred ammonia reactant is anhydrous ammonia. Aqueous ammonia may be employed, if desired, but is not preferred since it has been found to encourage the formation of by-products, such as fluorophthalic acids.

The process is preferably carried out at about atmospheric pressure, although super-atmospheric pressures may be employed, if desired. Generally, subatmospheric pressures are avoided, due to the volatility of the ammonia reactant.

It is preferred to carry out the ammonolysis reaction in a suitable solvent, particularly a solvent that will dissolve the fluorophthalic anhydride and that is substantially unreactive with ammonia. Suitable solvents include, for example, acetonitrile, methylene chloride, low molecular weight methoxy ethyleneglycols, such as dimethoxyethane and dimethoxy diethyleneglycol.

In accordance with this invention ammonium fluorophthalamates of the formula

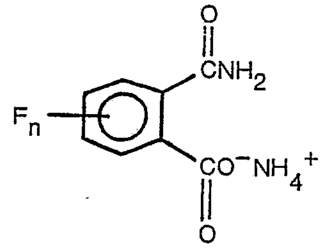

wherein n is 1 or 2 are formed. Typical compounds formed include ammoniumn 4,5-difluorophthalamate, ammonium 4-fluorophthalamate, ammonium 5-fluorophthalamate, ammonium 3-fluorophthalamate and ammonium 6-fluorophthalamate.

The ammonium fluorophthalamates of this invention are particularly useful as novel reactants in a novel and advantageous method for the preparation of fluoroanthranilic acids and fluoroanilines.

Fluoroanthranilic acids and fluoroanilines may be prepared, as described and claimed in copending EPC Application 81306177.7 (EP-A-55629), by

1) reacting an ammonium fluorophthalamate of the formula

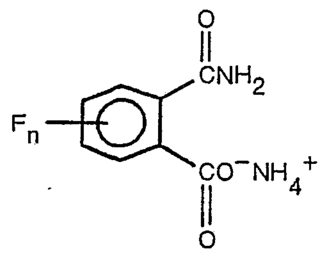

wherein n is 1 or 2, with an alkali metal or alkaline earth metal hypochlorite to form the corresponding fluoroanthranilic acid; and

2) decarboxylating the fluoroanthranilic acid by reaction with a mineral acid to form the corresponding fluoroaniline of the formula

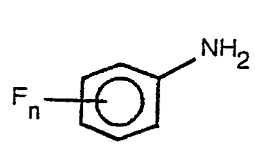

where n is as previously defined.

The fluoroanthranilic acid produced in step 1) above may be recovered separately. The fluoroanthranilic acids are useful intermediates in the preparation of various other fluorinated aromatic compounds. For example, the fluoroanthranilic acid may be reacted in an acidic medium such as hydrochloric acid with sodium nitrite to prepare fluorobenzoic acids.

(C) The ammonium fluorophthalamate may be converted to the corresponding fluorophthalamic acid by acidification. Typically, the fluorophthalamate is dissolved in a minimum amount of water and the solution is acidified to a pH of 1.5 to 6.0 by addition of an acid such as HCl, $H_2SO_4$, $H_3PO_4$, HBr, HI, HF or $HNO_3$. The fluorophthalamic acid may then be recovered by precipitation or by removal of the water, e.g. by low temperature evaporation or distillation.

The fluorophthalamic acids formed by the invention are characterized by the formula

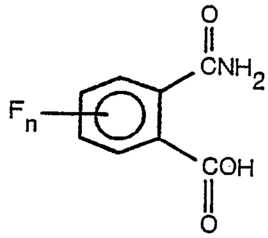

wherein n is 1 or 2 and typically include compounds such as 4,5-difluorophthalamic acid, 4-difluorophthalamic acid, 5-fluorophthalamic acid, 3-fluorophthalamic acid and 6-fluorophthalamic acid.

The fluorophthalamic acids of this invention are particularly useful as novel reactants in a novel and advantageous method for the preparation of fluoroanthranilic acids and fluoroanilines. The fluoroanthranilic acids and fluoroanilines may be prepared, as described and claimed in copending EPC Application 81306177.7 (EP-A-55629), by reacting a fluorophthalamic acid of the formula

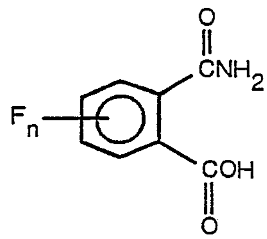

where n is 1 or 2, with an alkali metal or alkaline earth metal hypochlorite to form the corresponding fluoroanthranilic acids, and

decarboxylating the fluoroanthranilic acid by reaction with a mineral acid to form the corresponding fluoroaniline of the formula

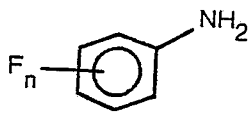

where n is as previously defined.

The fluoroanthranilic acids are useful intermediates in the preparation of various other fluorinated aromatic compounds. For example, the fluoroanthranilic acid may be reacted in an acidic medium such as hydrochloric acid with sodium nitrite to prepare fluorobenzoic acids.

(D) The fluorophthalamic acids may be dehydrated by moderate heating, typically at about 40° to about 350° Celsius to form the corresponding fluorophthalimides. Alternatively, the fluorophthalimides may be prepared directly from the ammonium salt of fluorophthalamic acids by heating to an elevated temperature, such as 150° to 350° Celsius.

The fluorophthalimides are characterized by the formula

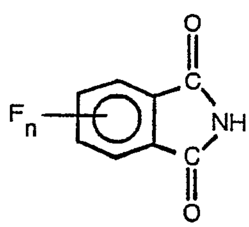 ,

wherein n is 1 or 2 and typically include compounds such as 4,5-difluorophthalimide and 3-fluorophthalimide.

(E) The fluorophthalimides may be converted to the salts thereof, especially the alkali metal salts, by reaction with a base, such as alkali metal base, such as potassium hydroxide.

The alkali metal salts of fluorophthalimides are characterized by the formula

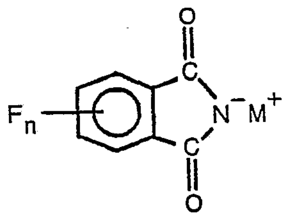

,

wherein n is 1 or 2 and m is an alkali metal ion. One typical compound is the potassium salt of 3-fluorophthalimide.

The fluorophthalimides and salts thereof have been found useful as intermediates in the preparation of various fluorinated aromatic compounds, especially in the preparation of fluoroanthranilic acids. The fluoroanthranilic acids are prepared by reacting a fluorophthalimide of the formula

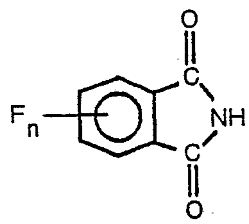

,

wherein n is 1 or 2, or an alkali metal salt thereof, with an alkali or alkaline earth metal hypochlorite, preferably sodium hypochlorite, to form a fluoroanthranilic acid of the formula

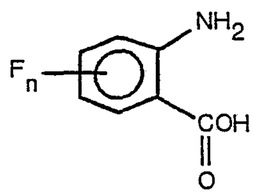

wherein n is as previously defined. The resulting fluoroanthranilic acid can if desired be decarboxylated with a mineral acid to provide a fluoroaniline of the formula

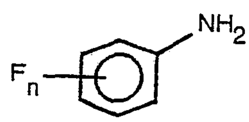

where n is 1 or 2.

The following specific examples are provided to further illustrate this invention and the manner in which it may be carried out. It will be understood, however, that the specific details given in the examples have been chosen for purpose of illustration and are not to be construed as a limitation on the invention. In the examples, unless otherwise indicated, all parts and percentages are by weight and all temperatures are in degrees Celsius.

Example 1

A) A mixture of 58.0 parts of anhydrous potassium fluoride, 21.7 parts of 4,5-dichlorophthalic anhydride, and 2.0 parts of Carbowax® m-peg 2000 catalyst was charged to a reactor, heated to about 175°C, and maintained thereat for about 54 hours. The product was removed from the reaction mixture by vacuum distillation and purified by recrystallization from a chloroform-hexane mixture to yield 12.15 parts of 4,5-difluorophthalic anhydride (66% yield) having a melting point of about 94°—96°C.

B) Acetonitrile (117.5 parts) was charged to a reaction vessel and anhydrous ammonia was bubbled in until the acetonitrile was saturated. The flow of ammonia was continued while a solution of 5.52 parts of the 4,5-difluorophthalic anhydride in 23.5 parts of acetonitrile was added slowly over a 15 minute period. A white precipitate formed. The reaction mixture was stirred for an additional 30 minutes. The acetonitrile was then removed by vacuum distillation to yield 6.1 parts of ammonium salt of 4,5-difluorophthalamic acid.

C) A portion of the ammonium salt was dissolved in a minimum amount of water, acidified by addition of concentrated hydrochloric acid to a pH of about 2.0, and re-crystallized from water. Analysis of the final product by $C^{13}$ nuclear magnetic resonance and infra-red spectroscopy confirmed it to be 4,5-difluorophthalamic acid.

D) One part of the 4,5-difluorophthalamic acid was heated to 170°C for a period of 30 minutes, then

cooled, dissolved in toluene and crystallized therefrom. The crystalline product was separated by filtration to yield 0.56 parts of 4,5-difluorophthalimide, having a melting range of 154°C—156° C. The structure was confirmed by $C^{13}$ nuclear magnetic resonance analysis.

E) To a solution of 0.18 parts of sodium hydroxide in 5.0 parts of water was added 0.4 parts of 4,5-difluorophthalimide. The solution was heated to about 50°C and 3.25 parts of 5.5% sodium hypochlorite solution was added. The reaction solution was heated to about 65°—70°C and maintained at that temperature range for about 30 minutes then cooled to about 20°—25°C. Concentrated hydrochloric acid was slowly added with the resultant formation of a precipitate. The addition of hydrochloric acid was continued until no further precipitate formed. The mixture was then extracted with chloroform, dried over anhydrous sodium sulfate, filtered. The remaining chloroform was removed under reduced pressure to yield 0.35 parts of 4,5-difluoroanthranilic acid having a melting point of 183°—183.5°C, and representing a yield of 92% based on the starting 4,5-difluorophthalimide.

F) A solution of 0.8 parts of 4,5-difluoroanthranilic acid in 40 parts of 1N sulfuric acid was placed in a reaction vessel equipped with a reflux condenser. The reaction solution was refluxed for 70 hours, then cooled, basified to a pH of about 8.0—9.0 by addition of 1N sodium hydroxide, saturated with sodium chloride and extracted with diethyl ether. The mixture was then dried over anhydrous sodium sulfate, filtered, and the diethyl ether removed under reduced pressure to yield 0.54 parts of product. Chromatographic analysis of the product indicated a 78% yield (based on the 4,5-difluoroanthranilic acid) of 96% pure, 3,4-difluoroaniline. The structure of the 3,4-difluoroaniline product was confirmed by $C^{13}$ NMR.

Example 2

A) A first solution of 3.32 parts of 3-fluorophthalic anhydride in 43 parts of dimethoxyethane was prepared. Separately, 86 parts of dimethoxyethane was added to a reaction vessel and ammonia was bubbled in to form a saturated solution of ammonia in dimethoxyethane. The ammonia addition was continued to maintain an excess while the 3-fluorophthalic anhydride solution was added to the ammonia/dimethoxyethane solution, slowly with stirring over a 40 minute period. When all of the 3-fluorophthalic anhydride had been added, the ammonia addition was stopped and the reaction mixture was stirred for an additional five minute period to assure completion of the reaction. The dimethoxyethane was removed under reduced pressure. The remaining crude product was analyzed by $C^{13}$ nuclear magnetic resonance and found to contain about 75% of the ammonium salt of 3-fluorophthalamic acid.

B) Following the procedure of Example 1C, a portion of the salt was dissolved in water, acidified, with concentrated hydrochloric acid to a pH of about 2.0, then cooled to form a white crystalline 3-fluorophthalamic acid having a melting point of 129°—132°C.

C) Following the procedure of Example 1D a portion of 3-fluorophthalamic acid is dehydrated by heating at about 170°C for about 30 minutes to form 3-fluorophthalimide.

D) Following the general procedure of Example 1E, a portion of 3-fluorophthalimide was reacted in an aqueous solution of sodium hydroxide and sodium hypochlorite in a 2:1 molar ratio, to form a mixture of 3- and 6- fluoroanthranilic acid.

Example 3

A) A mixture of 58.0 parts of anhydrous potassium fluoride, 21.7 parts of 4,5-dichlorophthalic anhydride and 0.5 parts of 18-crown-6, crown ether catalyst, was charged to a reactor and heated at about 150°C for about 6.5 hours. The product was separated from the reaction mixture and recrystallized as in Example 1A to yield 11.6 parts of 4,5-difluorophthalic anhydride (63% yield) having a melting range of about 94°—96°C.

B) Following the procedure of Example 1B, the 4,5-difluorophthalic anhydride product was reacted with ammonia to form the ammonium 4,5-difluorophthalamate.

Example 4

A) A mixture of 20 parts of 3-chlorophthalic anhydride, and 20 parts of anhydrous potassium fluoride was heated and maintained at about 235°C for about 9 hours. The reaction mixture was then cooled and the crude product removed by vacuum distillation and recrystallized from chloroform to yield 12.65 parts of purified 3-fluorophthalic anhydride (69% yield).

B) Ten parts of the 3-fluorophthalic anhydride was dissolved in 78.3 parts of acetonitrile and ammonia was bubbled into the solution until no 3-fluorophthalic anhydride could be detected (by thin layer chromatography on silica gel with a 7:2:1 mixture of toluene:ethyl acetate:acetic acid). The acetonitrile was then removed under reduced pressure to yield 14.8 parts of white solid — a mixture of the ammonium salts of 3- and 6-fluorophthalamic acid.

Example 5

A mixture of 20 parts of anhydrous potassium fluoride, 20 parts of 4-chlorophthalic anhydride and 2 parts of Carbowax® m-peg 2000 catalyst was charged to a reaction vessel and heated at 220°C for about 18 hours. The product was removed by distillation, then recrystallized from a chloroform-hexane solution to yield 8.13 parts of 4-fluorophthalic anhydride having a melting point of 76—78°C. Following the procedure

of Example 3B, the fluorophthalic anhydride is reacted with ammonia to yield the ammonium salts of a mixed 4- and 5-fluorophthalamic acid. The mixed salts are then acidified to yield a mixture of 4-fluorophthalamic acid and 5-fluorophthalamic acid.

Example 6

Dimethoxyethane (43 parts) was charged to a reactor and ammonia was bubbled in to form a saturated solution. The ammonia addition was continued while a solution of 1.84 parts of 4,5-difluorophthalic anhydride in 13 parts of dimethoxyethane was added slowly over a period of 0.5 hours. The reaction mixture was stirred for an additional 4 minute period and the dimethoxyethane was removed by vacuum distillation. The remaining white solid was the ammonium salt of 4,5-difluorophthalamic acid.

Example 7

A reaction mixture was prepared by mixing and grinding together 21.7 parts of 4,5-dichlorophthalic anhydride and 58 parts of anhydrous potassium fluoride. The mixture was charged to a reaction vessel together with 2 parts of Carbowax® MPEG 2000 catalyst, heated to about 210°C and maintained thereat for about 6.5 hours. The mixture was then cooled to about 25°C and allowed to stand for about 16 hours, then reheated and maintained at about 210°C for 7.5 hours. The reaction product was purified by vacuum distillation to yield 2.43 parts of product. Analysis of the product by $C^{13}$ nuclear magnetic resonance and infra-red analysis confirmed it to be 4,5-difluorophthaloyl fluoride.

Example 8

Following the procedure of Example 1C, a portion of the ammonium salt product of Example 4 was acidified and the resulting fluorophthalamic acid mixture was analyzed by $C^{13}$ nuclear magnetic resonance techniques. The acidified product was a mixture of 3-fluorophthalamic acid, 6-fluorophthalamic acid, and 3-fluorophthalic anhydride in a ratio of 71:17:12.

Example 9

A) A mixture of 10 parts of 3-fluorophthalic anhydride and 10 parts of concentrated ammonium hydroxide was heated to 210°C for about 2 hours. The crude reaction product consisted of 9.77 parts of solid having a melting point of 174.5-177°C. Analysis by H' nuclear magnetic resonance, confirmed the structure to be that of 3-fluorophthalimide;

B) The 3-fluorophthalimide product was dissolved in 160 parts of hot ethanol. The ethanol solution was added to a solution of 3.36 parts potassium hydroxide, 4 parts of water and 12 parts of ethanol. A yellow precipitate formed immediately. The mixture was cooled and filtered and the filter cake was boiled in acetone and filtered to yield 9.91 parts of potassium salt of 3-fluorophthalimide.

Example 10

A) A portion of the potassium salt of 3-fluorophthalimide (1.82 parts), prepared as in Example 9B, was dissolved in 26 parts of a solution of 0.77M NaOCl and 1.5M NaOH. The solution was heated and maintained at 80°C for about 1 hour, then cooled to about 20—25°C and acidified by addition of concentrated hydrochloric acid. The mixture was extracted with chloroform, dried over anhydrous sodium sulfate, filtered, and the chloroform removed under reduced pressure to yield 0.53 parts of a 95% pure mixture of 3-fluoroanthranilic acid and 6-fluoroanthranilic acid in a ratio of 58:37.

B) Following the general procedure of Example 1F, the mixture of 3- and 6-fluoroanthranilic acids is decarboxylated by reaction with 1N sulfuric acid, to yield a mixture of ortho- and meta-fluoroaniline.

**Claims**

1. A fluorophthalamic compound of the formula

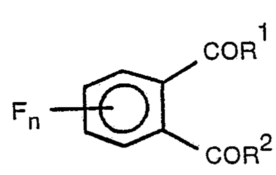

wherein n is 1 or 2, and $R^1$ is $NH_2$ and $R^2$ is —OH or —$ONH_4$ or $R^1$ and $R^2$ together are

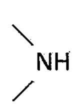 or 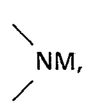,

wherein M is an alkali metal.

2. A process for preparing an ammonium salt of fluorophthalamic acid of the formula

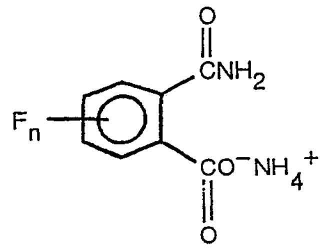

wherein n is 1 or 2, which process comprises

A) reacting a chlorophthalic anhydride of the formula

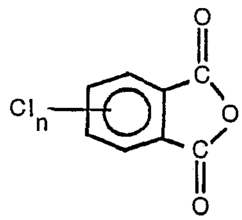

wherein n is 1 or 2, with potassium fluoride or cesium fluoride to form a fluorophthalic anhydride of the formula

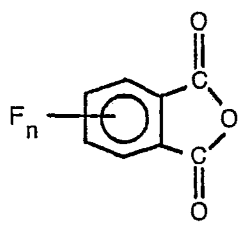

where n is 1 or 2; and

B) reacting the fluorophthalic anhydride with ammonia to form an ammonium salt of a fluorophthalamic acid.

3. A process for the preparation of a fluorophthalamic acid of the formula

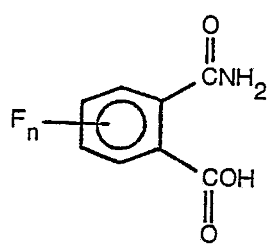

wherein n is 1 or 2 which comprises

C) acidifying an ammonium salt of a fluorophthalamic acid produced by the process of claim 2.

4. A process for the preparation of a fluorophthalimide of the formula

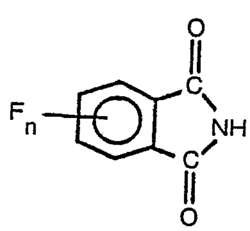,

wherein n is 1 or 2, which comprises

D) heating an ammonium salt of a fluorophthalamic acid produced by the process of claim 2 to a temperature of 150° to 350° Celsius, or

D') dehydrating a fluorophthalamic acid produced by the process of claim 3.

5. A process for preparing an alkali metal salt of a fluorophthalimide of the formula

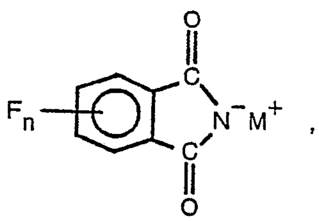

wherein n is 1 or 2, and M is an alkali metal ion, which process comprises

E) reacting a fluorophthalimide produced by the process of claim 4 with an alkali metal salt.

6. A process for the preparation of a fluoroanthranilic acid of the formula

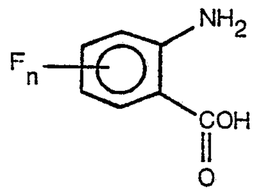

which process comprises reacting a fluorophthalimide of the formula

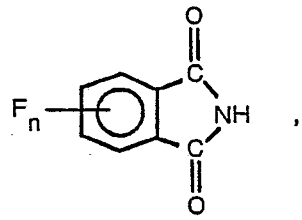

where n is 1 or 2, or an alkali metal salt thereof, with an alkali or alkaline earth metal hypochlorite.

7. A process for the preparation of a fluoroaniline of the formula

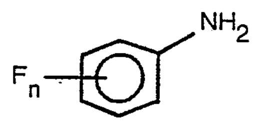

where n is 1 or 2 which process comprises reacting a fluorophthalimide of the formula

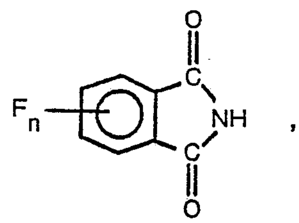

where n is 1 or 2, or an alkali metal salt thereof, with an alkali or alkaline earth metal hypochlorite and decarboxylating the resulting fluoroanthranilic acid with a mineral acid.

**Patentansprüche**

1. Fluorphthalaminverbindung der Formel

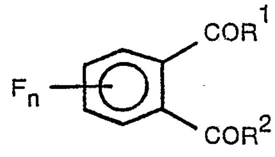

worin n gleich 1 oder 2 ist, und $R^1$ gleich $—NH_2$ ist und $R^2$ gleich $—OH$ oder $—ONH_4$ ist oder $R^1$ und $R^2$ zusammen gleich

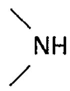 oder 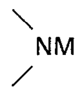

sind, worin M ein Alkalimetall ist.

2. Verfahren zur Herstellung eines Ammoniumsalzes von Fluorphthalaminsäure der Formel

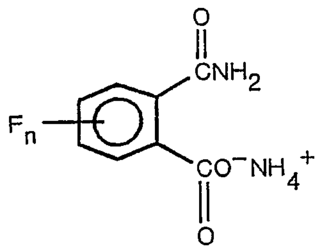

worin n gleich 1 oder 2 ist, wobei

A) ein Chlorphthalsäureanhydrid der Formel

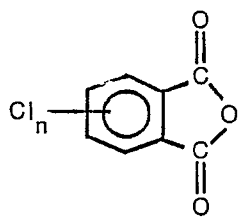

worin n gleich 1 oder 2 ist, mit Kaliumfluorid oder Cesiumfluorid umgesetzt wird, um ein Fluorphthalsäureanhydrid der Formel

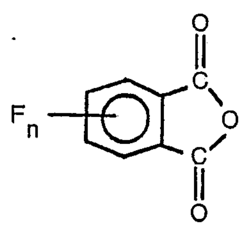

zu bilden, worin n gleich 1 oder 2 ist, und

B) das Fluorphthalsäureanhydrid mit Ammoniak umgesetzt wird, um ein Ammoniumsalz einer Fluorphthalaminsäure zu bilden.

3. Verfahren zur Herstellung einer Fluorphthalaminsäure der Formel

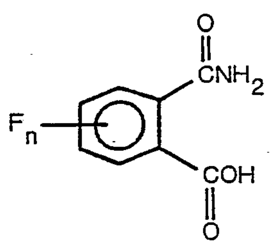

worin n gleich 1 oder 2 ist, wobei

C) ein Ammoniumsalz einer nach dem Verfahren gemäß Anspruch 2 hergestellten Fluorphthalaminsäure acidifiziert wird.

4. Verfahren zur Herstellung eines Fluorphthalimids der Formel

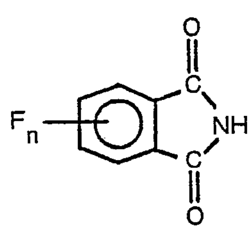 ,

worin n gleich 1 oder 2 ist, wobei

D) ein Ammoniumsalz einer Fluorphthalaminsäure, welches nach dem Verfahren gemäß Anspruch 2 hergestellt wurde, auf eine Temperatur von 150 bis 350°C erhitzt wird, oder

D') eine Fluorphthalaminsäure, die nach dem Verfahren gemäß Anspruch 3 hergestellt wurde, dehydratisiert wird.

5. Verfahren zur Herstellung eines Alkalimetallsalzes eines Fluorphthalimids der Formel

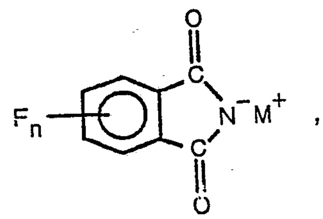

worin n gleich 1 oder 2 ist, und M ein Alkalimetallion ist, wobei

E) ein Fluorphthalimid, welches nach dem Verfahren gemäß Anspruch 4 hergestellt wurde, mit einem Alkalimetallsalz umgesetzt wird.

6. Verfahren zur Herstellung einer Fluoranthranilsäure der Formel

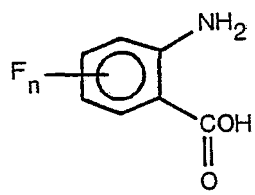

wobei ein Fluorphthalimid der Formel

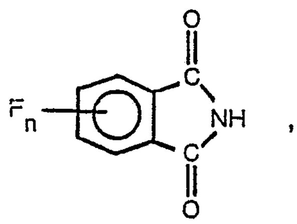

worin n gleich 1 oder 2 ist, oder ein Alkalimetallsalz davon, mit einem Alkali- oder Erdalkalimetallhypochlorit umgesetzt wird.

7. Verfahren zur Herstellung eines Fluoranilins der Formel

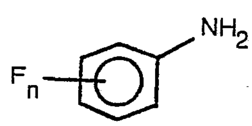

worin n gleich 1 oder 2 ist, wobei ein Fluornaphthalimid der Formel

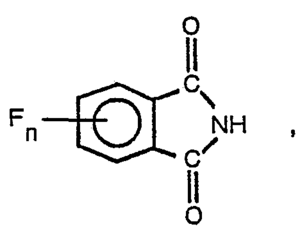

worin n gleich 1 oder 2 ist, oder ein Alkalimetallsalz davon, mit einem Alkali- oder Erdalkalimetallhypochlorit umgesetzt wird und die erhaltene Fluoranthranilsäure mit einer Mineralsäure decarboxyliert wird.

**Revendications**

1. Composé fluorophtalamique de la formule

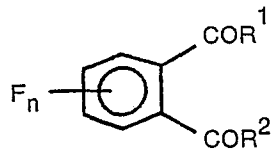

dans laquelle n est égal à l ou 2, et $R^1$ est $—NH_2$ et $R^2$ est —OH ou $—ONH_4$, ou $R^1$ et $R^2$ sont ensemble

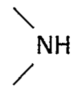 ou 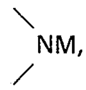

dans lequel M est un métal alcalin.

2. Procédé pour préparer un sel d'ammonium d'acide fluorophtalimique de la formule

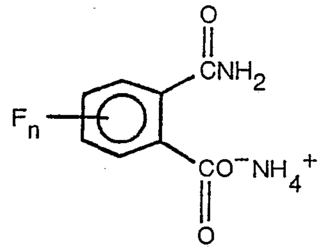

dans laquelle n est égal à 1 ou 2, lequel procédé consiste à

(A) faire réagir un anhydride chlorophtalique de la formule

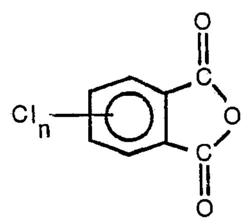

dans laquelle n est égal à 1 ou 2, avec du fluorure de potassium ou du fluorure de césium pour former un anhydride fluorophtalique de la formule

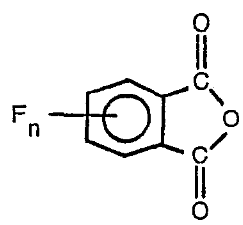

dans laquelle n est égal à 1 ou 2; et

(B) faire réagir l'anhydride fluorophtalique avec de l'ammoniac pour former un sel d'ammonium d'un acide fluorophtalamique.

3. Procédé pour préparer un acide fluorophtalamique de la formule

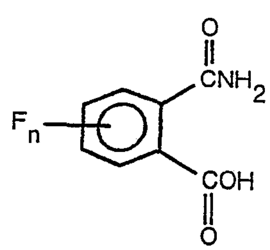

dans laquelle n est égal à 1 ou 2 en:

(C) acidifiant un fluorophtalamate d'ammonium obtenu par le procédé de la revendication 2.

4. Procédé pour préparer un fluorophtalimide de la formule

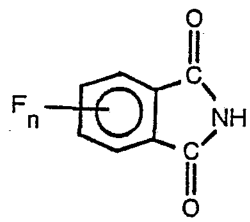

,

dans laquelle n est égal à 1 ou 2 en:

(D) chauffant un fluorophtalamate d'ammonium produit par le procédé de la revendication 2 à une température de 150° à 350°C, ou

(D') déshydratant un acide fluorophtalamique produit par le procédé de la revendication 3.

5. Procédé pour préparer un sel de métal alcalin d'un fluorophtalimide de la formule

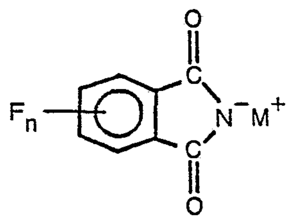

,

dans laquelle n est égal à 1 ou 2 et M est un ion de métal alcalin, dans lequel:

(E) on fait réagir un fluorophtalimide produit par le procédé de la revendication 4 avec un sel de métal alacalin.

6. Procédé pour préparer un acide fluoroanthranilique de la formule

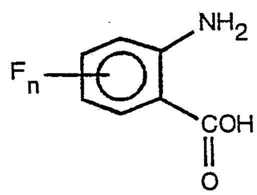

dans lequel on fait réagir un florophtalimide de la formule

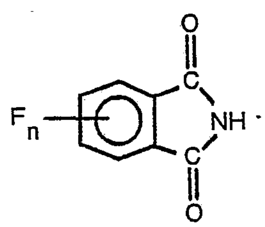

,

dans laquelle n est égal à 1 ou 2, ou un sel de métal alcalin de ce fluorophtalimide, avec un hypochlorite de métal alcalin ou alcalino-terreux.

7. Procédé pour préparer une fluoroaniline de la formule

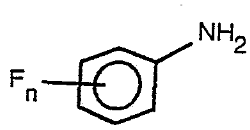

dans laquelle n est égal à 1 ou 2, dans lequel on fait réagir un fluorophtalimide de la formule

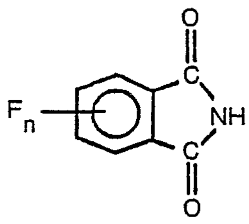

,

dans laquelle n est égal à 1 ou 2, ou un sel de métal alcalin de ce fluorophtalimide, avec un hypochlorite de métal alcalin ou alcalino-terreux et on décarboxyle l'acide fluoroanthranilique résultant avec un acide minéral.